**Europäisches Patentamt**

**European Patent Office**

**Office européen des brevets**

(19)

(11) Publication number: **0 065 390**
A1

# (12) EUROPEAN PATENT APPLICATION

(21) Application number: **82302325.4**

(22) Date of filing: **06.05.82**

(51) Int. Cl.³: **C 07 H 13/06**

(30) Priority: **11.05.81 GB 8114293**

(43) Date of publication of application: **24.11.82**
**Bulletin 82/47**

(84) Designated Contracting States: **BE DE FR IT LU NL**

(71) Applicant: **TATE & LYLE PUBLIC LIMITED COMPANY,**
**Sugar Quay Lower Thames Street, London, EC3R 6DQ**
**(GB)**

(72) Inventor: **James, Kenneth, 34, Crawford Close Earley,**
**Reading Berkshire (GB)**
Inventor: **Dolan, Charles Christopher, 25, Melville Road,**
**Bottle Lancashire (GB)**

(74) Representative: **Ruffles, Graham Keith et al, MARKS &**
**CLERK 57-60 Lincoln's Inn Fields, London WC2A 3LS**
**(GB)**

(54) Purification of sucrose esters.

(57) Sucrose ester-containing material obtained by base-catalysed transesterification of sucrose with a fatty acid ester is purified by a process comprises the steps of:

    (i) acidifying an aqueous dispersion of the material to between pH 4 and 7 to give an aqueous phase and an organic phase;

    (ii) at or above the melt temperature of the organic phase, recovering the organic phase;

    (iii) mixing the organic phase with ethyl acetate containing 0 to 4% water, to give at a temperature of 0 to 20°C a liquid phase and a solid phase; and

    (iv) recovering the solid phase as a sucrose ester-rich product.

EP 0 065 390 A1

0065390

M&C FOLIO: 41334                                    WANGDOC: 0047C

PURIFICATION OF SUCROSE ESTERS

The present invention relates to the purification of sucrose esters of fatty acids.

Sucrose esters of fatty acids (also known simply as "sucrose esters") are usually produced by a transesterification reaction in which sucrose is made to react with a fatty acid ester, typically either an alkyl ester of the fatty acid or a triglyceride.

Conventionally the transesterification has been carried out in a homogenous or quasi-homogenous reaction medium in the presence of a catalyst. For instance, there have been proposals to effect the reaction in a solvent (see, for example, USA patent No 3 714 144), or in a "transparent emulsion" (see, for example, USA patent No 3 480 616). In general, the catalyst is a basic material, with the alkali metal salts of weak acids being particuarly preferred. More recently, we have developed an alternative catalysed transesterification procedure which is based on the use of heterogenous conditions (see UK patent No 1 399 053 and No 1 499 989) and which gives a surfactant containing sucrose esters.

In practice, and despite the variety of available processes for manufacturing the sucrose esters of fatty acids, none

0065390

directly gives a sucrose ester with high purity.

Specifically, the available procedures give products which contain not only the mono-, di-and higher fatty acid esters of sucrose, but usually also any catalyst employed in the reaction, unreacted sucrose and/or starting ester, and by-products derived from the sucrose and/or starting ester. The typical product obtained directly from a transesterification reaction is thus a complex mixture of components.

Consider for example, the transesterification of sucrose with tallow, a naturally occurring triglyceride. Tallow in fact contains many triglycerides, reflecting the various fatty acids which are present to make up the fatty acid profile of tallow. For this example, the simplification is therefore made that the starting ester is glyceryl tristearate, that is, the main triglyceride in tallow. Then assuming that the catalyst is sodium methoxide, and ignoring the possible presence of solvent, emulsifier or other reaction aid, the typical product might contain sucrose monostearate, sucrose distearate, higher stearates of sucrose, unreacted sucrose, caramelized sucrose, unreacted glyceryl tristearate, glyceryl distearate, glyceryl monostearate, glycerol, methanol and sodium stearate.

While such impure products can be used directly as surfactants or for some other applications, there is a need for purification procedures which will give the sucrose mono- and di-esters of the fatty acid, either relatively pure or in

0065390

admixture as "sucroglycerides" with mono-, di- and triglycerides obtained when the starting ester is a triglyceride.

Thus, for example, there is an EEC ("European Economic Community") Specification No E473 which lays down a standard for sucrose esters of food fatty acids and which can be summarized as shown in the following Table 1. Similarly, for sucroglycerides there is EEC Specification No E474, also summarized in Table 1. The amounts shown in Table 1 are in weight percentages.

Table 1

| Component | E 473 | E474 |
|---|---|---|
|  | Sucrose Esters | Sucroglycerides |
| mono- and diesters of sucrose with edible fatty acids | 80 or more | 40 to 60 |
| glycerides | 20 or less | 40 to 60 |
| free sucrose | 5 or less | 5 or less |
| free fatty acid | 3 or less | 3 or less |
| sulphated ash | 2 or less | 2 or less |
| residual permitted solvent | as specified | as specified |

There is a set WHO/FAO specification (FAO Food and Nutrition
Paper No 4 (1978)) giving a recommended procedure for
analysing sucrose ester products. The application of this
standard procedure to some sucrose ester products suggests
that in the past there has been a certain optimism on
purity. Indeed, if one reconsiders the literature on the
production and purification of sucrose esters, it is possible to see that many statments on yield and purity are of doubtful validity. The WHO/FAO procedure is itself based on Bertrand's assay and is partly empirical in that an adjusting factor of 1/0.503 is applied to the analytical data. Thus even this analytical method is not free from criticism. Put simply, analytical techniques such as gas chromatography are still being improved to permit a more realistic appraisal of the purity of sucrose ester products.

Some purification procedures have been developed which might enable one to purify crude sucrose esters and obtain a product in accordance with a desired specification, for example the EEC Specification No E473 or E474.

However, while these known procedures work on a laboratory scale, we find that there are sometimes difficulties in reducing sufficiently the level of free sucrose, sucrose-derived coloured matter and other water-soluble impurities in order to give a product conforming with a given product specification.

In accordance with the present invention we provide a process for purifying a sucrose ester-containing material obtained by base-catalysed transesterification of sucrose with a fatty acid ester, the fatty acid profile preferably including at least 50% of one or more acids with 16 or more carbon atoms and preferably including 5 to 50% of one or more acids with unsaturation, which process comprises the steps of:

(i)     acidifying an aqueous dispersion of the material to between pH 4 and 7 to give an aqueous phase and an organic phase;

(ii)    at or above the melt temperature of the organic phase, recovering the organic phase;

(iii)   mixing the organic phase with ethyl acetate containing 0 to 4% water, to give at a temperature of 0 to 20°C a liquid phase and a solid phase; and

(iv)    recovering the solid phase as a sucrose ester-rich product.

By the adoption of the present process it is readily possible to remove appreciable proportions of soaps and other impurities from a crude sucrose ester.  In particular, it is now possible to take crude products produced in accordance with the process of UK patent No 1339053 and obtain on an industrial scale sucrose esters or sucroglycerides meeting the respective EEC Specification No E473 or E474.

The present invention is applicable not only to crude sucrose ester products produced in accordance with the process of UK patent No 1399053, but also to other sucrose ester-containing materials.  The process can be used for example on other crude products derived from triglycerides as well as on crude products derived from alkyl esters, and on partially purified products.  For instance the process can be employed with sucrose-ester products obtained using a process in accordance with the process of UK patent No 1399053 which have then been partially purified by extraction of glycerides and other
extractable material using a solvent such as ethyl acetate.

The present process is generally applicable to the
purification of sucrose esters of fatty acids. In practice
the process is easier to operate if at least 50% of the acids
are higher fatty acids (having 16 or more carbon atoms). It
is also desirable that 5 to 50%, more usually 20 to 45%, of
the acids are unsaturated. However, the process can readily
be made to work with other fatty acid esters, such as those
of coconut oil, provided that certain elementary precautions
are taken in handling the acidified dispersion at step (i)
and the organic phase at steps (ii) and (iii). In
particular, the acidified dispersion has a tendency to
emulsify, while in steps (ii) and (iii) some cooling may be
needed.

Examples of sucrose esters with which the process can be
performed include those with oleic, stearic, tallow, coconut,
palm, palm kernel and other single or mixed fatty acids. The
lauric and other lower esters have greater water solubility,
and a lower melting point, but nevertheless the present
process can be used with such materials.

In the present process, an aqueous dispersion of the material
to be purified is acidified at step (i). The solution
suitably contains 5 to 15% of the material, and is preferably
at below the melt temperature subsequently specified for step
(ii). Thus, for a tallow-derived sucrose ester material produced by the process of UK patent No 1399053, the solution is preferably held at 20 to 40°C for the addition of acid.

For preference the acid is a concentrated inorganic or organic acid, with examples including glacial acetic acid, concentrated sulphuric, hydrochloric, or nitric acid. Sufficient acid is added to give a pH of 4 to 7, more usually around pH 4.5 with weak organic acids or approaching pH 7 with strong mineral acids. The acidification converts soaps to the corresponding free fatty acids.

The free fatty acids, especially the higher fatty acids, do not have appreciable solubility in water, and form an organic phase with the sucrose esters and other organic matter. If the temperature is below the melt temperature the organic phase usually takes the form of a floc suspended in the aqueous phase. The aqueous phase will contain most of the sucrose present in the crude material and lesser amounts of the other components, along with salts resulting from the acidification.

In the step (ii), the organic phase obtained in step (i) is recovered at or above the melt temperature of the organic phase. Thus, gentle heating of the system results in melting of the free fatty acids, with a temperature of about 50°C being appropriate for the tallow-derived crude material mentioned above. The melted organic phase can then be separated off, and further purified if desired. For example, the organic phase can be mixed with cold water, warmed to give separate liquid phases, and the organic phase separated off and evaporated to remove water. The organic phase can be drawn off in simple manner where there is good phase separation, though filtering or centrifuging can be employed if needed.

The recovered organic phase is typically a semi-viscous mobile oil which sets on standing at room temperature. It is essentially free of sucrose, inorganic salts, soaps and added acid, and containing less than 1.5% water. For convenience, the oil can be held at up to 80°C to enhance mobility.

In the step (iii), the oil representing the recovered organic phase is mixed with ethyl acetate containing 0 to 4% water: the ethyl acetate can be dry or nearly dry. Suitably the oil: ethyl acetate ratio is between 1:10 and 1:3, expressed as kg: $dm^3$, for example from 1:5 to 1:7. With the mixture at 0 to 20°C, a solid phase is obtained which is rich in sucrose esters, together with a liquid phase based on the ethyl acetate and typically containing free fatty acids and some of the initial fatty acid ester (alkyl ester or triglyceride), as well as by-products formed from the initial ester. One or more extractions with the ethyl acetate can be used, preferably two to four extractions using a higher temperature at each extraction. The temperature for at least the first extraction is in the range 0 to 20°C

In the step (iv), the solid phase is recovered, usually by centrifuging. If desired, the recovered solid phase can be further purified. For example, it can be mixed with further ethyl acetate, and the resultant solid phase dried under vacuum.

By adoption of the present process, it is now possible to take a crude brown mixture resulting from a triglyceride transesterification and obtain an off-white free-flowing powder which meets the EEC Specification No E473 for sucrose esters.

The present invention is illustrated by the following non-limiting examples.

In the examples the following abbreviations are employed:

| G | glycerol |
| So | soap |
| Su | sugar |
| Mg | monoglyceride |
| Me | monoester |
| Dg | diglyceride |
| De | diester |
| Tg | triglyceride |
| FFA | free fatty acid |
| Is | inorganic salts |

The analyses were performed using gas chromatography after silylation. The esters are calculated on dominant single ester peaks. For example, tallow monoesters are estimated on the basis of sucrose monostearate using calibrated response factors.

The use of gas chromatography allows rapid and relatively accurate analysis of the complex mixtures. However, preferential extraction may lead to some error in the analyses (for example if other esters are extracted in preference to sucrose monostearate, thereby altering the fatty aicd profile of tallow). As such, the analyses do not total 100%. Furthermore, the gas chromatography does not detect the inorganic salts (which do not silylate), again explaining why the analyses do not total 100%.

For each example the material to be purified was obtained by transesterification of sucrose with tallow, using a process in accordance with UK patent No 1399053. In general the unpurified material was tan brown in colour.

## Example 1

1 kg of crude tallow-derived sucrose ester material with the following analysis was purified:
..

| G | So | Su | MG | ME | DG | DE | TG | TOTAL% |
|---|----|----|----|----|----|----|----|--------|
| 1.9 | 28.3 | 21.0 | 13.2 | 16.2 | 4.3 | 7.8 | 1.3 | 94 |

The crude material was dispersed in 6 dm$^3$ of water at room

12    0065390

temperature and 183 g glacial acetic acid in 175 $cm^3$ water
was added in portions.  The acidified mixture was stirred for
1/2 hour and then warmed to 50°C which caused the organic
phase to melt.  The lower aqueous layer was removed and 6
dm  process water added to the melt.  The mixture was
stirred and the temperature once again brought to 50°C.  The
lower aqueous layer was removed and a further 6 $dm^3$ process
water added to the solid.  The temperature was brought to
90°C and the system gently stirred for 0.75 hour.  The turbid
lower layer was removed, leaving an organic phase which was
dried to an oil, weighed and analysed.

| G | FFA | Su | MG | ME | DG | DE | TG | TOTAL% |
|---|-----|-----|------|------|-----|------|-----|--------|
| – | 37.8 | 0.4 | 15.0 | 24.1 | 7.3 | 12.2 | 1.8 | 97.9 |

The weight of dried oil was 656.3 g, representing a 99.3%
recovery of sucrose esters at this first stage.

617 g of the dried oil was then extracted with three portions
of dry ethyl acetate, reducing the volume/solid ratio of each
extraction from an initial ratio of 6:1.  The temperatures of
the extractions were 5°C, 9°C, 17.5 to 20°C.  In this and the
Examples 2 and 3 the extraction mixture was left for half an
hour before separating the phases.

The ethyl acetate was removed by evaporation and the extracts
dried, weighed and analysed:

13

0065390

| Extract | Weight | FFA | Su | MG | ME | DG | DE | TG |
|---------|--------|-----|-----|------|------|------|------|-----|
| 1* | 362.0g | 48.2 | 2.7 | 17.3 | 5.5 | 10.0 | 6.0 | 2.4 |
| 2 | 65.2g | 43.6 | 1.2 | 13.3 | 9.7 | 9.2 | 11.4 | 2.7 |
| 3 | 31.4g | 28.8 | - | 13.4 | 22.9 | 6.9 | 24.2 | 3.5 |

* A small amount of solid material passed through the filter during this extraction.

The extracted esters were dried on a Büchi rotary evaporator and then overnight in a vacuum oven at 60°C. The dried material was ground to a creamy white solid which gave the following analysis.

| G | So | FFA | Su | MG | ME | DG | DE | TG | TOTAL% |
|-----|-----|-----|-----|-----|------|-----|------|-----|--------|
| - | 2.9 | 2.2 | 0.6 | 1.9 | 74.1 | 1.2 | 26.7 | 0.2 | 109.8 |

The weight of product was 160.7g, corresponding to a 72.2% yield based on the initial ester content.

## Example 2

1 kg of tallow-derived material with the following analysis was dispersed in 10 dm$^3$ water.

| G | So | Su | MG | ME | DG | DE | TG | TOTAL |
|-----|------|------|------|------|-----|-----|-----|-------|
| 1.6 | 30.2 | 20.2 | 13.3 | 15.8 | 8.7 | 8.1 | 2.2 | 99.1 |

183 glacial acid in 175 cm$^3$ water acid was then added portionwise, with stirring. The mixture was allowed to react for 1/2 hour and then heated to 50°C. The insoluble material coalesced and the lower aqueous layer was removed.

The crude FFA-containing mixture was then made up to 10 dm$^3$ with process water, stirred and heated to 50°C. Coagulation occurred and the liquor was removed.

This treatment with process water was repeated.

The total amount of solids removed by the three washings was 380.5g of which 193.4g was sucrose. Ester losses, due to acidic hydrolysis of the glycoside linkage and/or dissolution of the esters in the wash water, were negligible.

The wet mixture was then dried on a Büchi evaporator and in a vacuum oven at 60°C overnight.

The dried oil amounted to 679.6g and had the following analysis:

| G | So | FFA | Su | MG | ME | DG | DE | TG | TOTAL% |
|---|----|-----|----|----|----|----|----|----|--------|
| - | 0.4 | 34.6 | - | 17.9 | 22.7 | 11.7 | 11.9 | 1.6 | 101.0 |

The total sucrose ester content was thus 235.1g, which represents a 99.6% recovery at this stage. The dried oil was then extracted with three portions of dry ethyl acetate as in Example 1. The three extractions were all performed at below 10°C, the average being 7°C. The extract of esters was dried, powdered and analysed.

| G | So | FFA | Su | MG | ME | DG | DE | TG | TOTAL% |
|---|----|-----|----|----|----|----|----|----|--------|
| - | 4.4 | 3.7 | 0.9 | 2.8 | 63.8 | 2.3 | 26.9 | 0.3 | 105.1 |

The weight of product was 165.1g, representing 70.2% yield based on the initial sucrose ester content.

## Example 3

183g glacial acetic acid in 175 $dm^3$ water was added portionwise with stirring and at room temperature to a dispersion of 1 kg of crude tallow-derived material in 6 $dm^3$ water, the material having the following analysis:

| G | So | Su | MG | ME | DG | DE | TG | TOTAL% |
|---|----|----|----|----|----|----|----|--------|
| 1.9 | 28.3 | 21.0 | 13.2 | 16.2 | 4.3 | 7.8 | 1.3 | 94.0 |

When the addition was complete the mixture was stirred at room temperature for half an hour. The temperature was raised to 50°C and the lower aqueous layer removed.

The mixture was reconstituted with water to its original volume, heated to 50°C and the aqueous layer was again removed. The solid was once again made up to its original volume with water and then heated to 75°C for half an hour.

The turbid lower layer was removed, evaporated and analysed. The analysis showed that this final washing contained 1% of the total esters together with fatty acids and mixed glycerides, indicating that they were present as an emulsion.

The dried oil was extracted twice with 6 volumes of cold dry ethyl acetate to produce esters which gave the following analysis:

| G | So | FFA | Su | MG | ME | DG | DE | TG | TOTAL% |
|---|----|-----|-----|-----|-----|-----|-----|-----|--------|
| 0 | 2.0 | 2.0 | 1.9 | 1.4 | 76.8 | 0.4 | 16.8 | 0.1 | 101.4 |

## Example 4

A large scale purification was carried out on material with the analysis shown in Table 2:

TABLE 2

|  | Kilograms | Dry wt% |
|---|---|---|
| G | 1.6 | 1.6 |
| So | 26.0 | 26.0 |
| Su | 22.3 | 22.3 |
| MG | 10.5 | 10.5 |
| ME | 14.8 | 14.8 |
| DG | 8.7 | 8.7 |
| DE | 8.9 | 8.9 |
| TG | 5.3 | 5.3 |
| IS | 1.9 | 1.9 |
| TOTAL | 100 | 100 |

The crude product thus contains 23.7% sucrose mono- and die-esters, 24.5 glycerides and 51.8% soaps, sucrose and other impurities.

100 kilograms of the crude material was mixed with 1000 kilograms of water at 25°C. 20 kilograms of 80% acetic acid was then added to the solution resulting in the production of a floc. On raising the temperature to 50°C, the floc separated as a molten upper layer. The lower, coloured aqueous layer was drained off.

The upper layer was then washed with 1000 kilograms of cold water giving a fine dispersion. On raising the temperature to 50°C, a separation of the phases occurred. After a period

0065390

of settling, the lower aqueous layer was drained off.

Cold water was then added in a volume approximately equal to
that of the remaining organic phase, and further washing
carried out under agitation.  The temperaturee was then
raised to 80°C to effect a separation of the phases, and the
lower aqueous layer again discarded.

As a result, a semi-viscous mobile oil was obtained.  The oil
was then passed through a wiped film evaporator to remove
water.  The evaporation was carried out umder partial vacuum
with a surface temperature of 140 to 170°C.

The resultant dry oil had the composition shown in the
following Table 3.

Table 3

|     | Kilograms | Dry wt% |
| --- | --- | --- |
| G   | 0.1   | 0.17 |
| FFA | 18.9  | 31.5 |
| Su  | 0.4   | 0.67 |
| MG  | 8.7   | 14.5 |
| ME  | 12.7  | 21.2 |
| DG  | 7.05  | 11.8 |
| DE  | 7.6   | 12.7 |
| TG  | 4.5   | 7.5 |
| IS  | -     | -   |
| $H_2O$ | 0.9 | -   |
| Total | 60.85 | 100 |

It will be seen that the sucrose content has been dramatically reduced with disproportionately lower losses of the other components.

The dry oil was then slowly added to 360 kilograms of ethyl acetate containing 2.5% by weight of water. The resulting mixture was agitated at 10 to 15°C and then centrifuged to give solids rich in sucrose esters.

The solids were then again mixed with 360 kilograms of ehtyl acetate containing 2.5% by weight of water.. The remaining solids were then separated by centrifuging and dried under vacuum with moderate heat.

The resultant product was an off-white free flowing powder having the composition shown in Table 4.

Table 4

|      | Kilograms | Dry wt% |
|------|-----------|---------|
| G    | 0.017     | 0.1     |
| FFA  | 0.38      | 2.3     |
| Su   | 0.42      | 2.5     |
| MG   | 0.53      | 3.2     |
| ME   | 10.36     | 62.4    |
| DG   | 0.68      | 4.1     |
| DE   | 4.03      | 24.3    |
| TG   | 0.18      | 1.1     |
| $H_2O$ | trace   | -       |
| Total | 16.6     | 100     |

The product thus contains 86.7% sucrose esters, 8.4% glycerides and 4.9% impurities.

Example 5

Example 1 was repeated but with variation in the ethyl acetate treatment.

When the intermediate dried oil was stirred with ethyl acetate containing 2-4% water at a temperature of 20°C and a concentration of from 9-19% (w/v) the amount of fatty acid retained in the insoluble (sucrose ester) phase was significantly less and the total recovery of esters was considerably more than when dry ethyl acetate was employed (see Table below). Although the products contained 7-9% fatty acid, the recovery of esters in the product (the residue after Büchi evaporation) and also the content of the esters therein were good.

## TABLE 5

| %SOLIDS IN ETOAc W/V | %$H_2O$ V/V | %RECOVERY ESTERS IN RESIDUE | | | % ESTERS IN RESIDUE | | %FA IN RESIDUE |
|---|---|---|---|---|---|---|---|
| | | ME | DE | ME+DE | ME | DE | |
| 18.7 | 4 | 82.6 | 60.5 | 76.3 | 65.3 | 19.4 | 9.2 |
| 9.05 | 2 | 80.5 | 46.5 | 70.7 | 73.5 | 17.1 | 6.7 |
| 9.4 | 0 | 51.8 | 28.8 | 45.2 | 69.6 | 15.7 | 12.5 |
| 18.8 | 0 | 46.0 | 39.7 | 44.2 | 50.2 | 17.5 | 22.4 |

Thus, for example, when 18.7% w/v of crude material was mixed with ethyl acetate containing 4% v/v of water at 18°C, 76.3% of the sucrose mono- and di-esters was recovered, with the monoester forming 65.3% of the residue and the di-ester forming 19.4% of the residue.  The residue further contained
9.2% of the fatty acid.

Of the results shown in Table 5, the best one is taken to be
that employing 9.05% solids in ethyl acetate at 2% water
content.  This experiment gave a residue rich in sucrose
esters, poor in fatty acid, and with good recovery of the
esters into the residue.

Example 6

A sample of crude sucrose ester material similar to that
employed in Example 4 was extracted with ethyl acetate to
remove glycerides.  The resultant partially purified material
had the following analysis.

| G | So | Su | MG | ME | DG | DE | TG | TOTAL% |
|---|----|----|----|----|----|----|----|--------|
| 1.6 | 35.0 | 26.6 | 2.12 | 19.8 | 0.3 | 9.0 | 0.8 | 95.2 |

200g of the partially purified material was dispersed in 2
dm$^3$ of water at room temperature and 31.4 g glacial acetic
acid was added in portions.  The acidified mixture was
stirred and warmed to 50°C which caused the organic phase to
coagulate.  The precipitate was washed by decanting and
filtered off.  The filtrate was dried to give 144.0g of dried
solid which had the following analysis.

23

0065390

| G | FFA | Su | MG | ME | DG | DE | TG | TOTAL% |
|---|-----|-----|-----|------|-----|-----|----|--------|
| 0 | 47.2 | 2.6 | 2.2 | 23.7 | 2.8 | 9.5 | 0 | 88.0 |

The recovery was 83% of sucrose esters at this first stage.

The dried material was then extracted with three portions of dry ethyl acetate, reducing the volume/solid ratio of each extraction from an initial ratio of 6:1. In this and the Examples 2 and 3 the extraction mixture was left for half an hour before separating the phases.

The ethyl acetate was removed by evaporation and the extract dried, weighed and analysed. 41.6g of dried product was obtained.

| G | So | FFA | Su | MG | ME | DG | DE | TG | TOTAL% |
|-----|------|-----|-----|-----|------|-----|------|----|--------|
| 0.2 | 10.8 | 4.9 | 7.8 | 1.3 | 70.5 | 0 | 24.0 | 0 | 119.5 |

Following on from these examples concerned with tallow-derived material, further work was carried out to demonstrate the general applicability of the process. This work forms the basis of the following examples.

Example 7

Preparation of material to be purified

Scale

|  | weight (g) |
|---|---|
| Methyl tallowate | 1114 |
| Potassium hydroxide | 45.4 |
| Potassium carbonate | 113.4 |
| Sugar (powdered) | 726 |

The methyl tallowate was stirred in an oil bath and potassium soaps were prepared by adding potassium hydroxide and homogenising. The potassium carbonate was added and the mixture stirred for 0.5 hour, while the temperature was raised to 125°C. The sugar was added in increments of 125g over 1.5 hours and the mixture was then allowed to react for 18 hours.

Weight of crude material       1870.3g

Analysis of crude material

| G | So | Su | MG | ME | DG | DE | TG | TOTAL% |
|---|---|---|---|---|---|---|---|---|
| 0 | 47.0 | 30.4 | 0 | 15.8 | 0 | 6.6 | 0 | 99.8 |

<u>Isolation of tallow sucrose esters</u>

<u>Scale:</u>

     1kg product + 6dm$^3$ water

     200cm$^3$ glacial acetic acid

The product was dispersed in water and the acetic acid added slowly, with stirring, at room temperature. The mixture was stirred for 0.5 hour, and the temperature slowly raised. The material coagulated at 23°C and melted at 30°C. The lower aqueous layer was removed and the oil was washed twice by heating with water and decantation. The organic material was then dried using Büchi rotary vacuum equipment.

Weight of organic material   633.6g

The organic material was then extracted two times using ethyl acetate in respective amounts at respective temperatures and for respective times of 4dm$^3$, 5°C and 30 minutes; and 3dm$^3$, 7°C and 30 minutes. 99.5g of purified product with the following analysis was obtained.

| G | FFA | Su | MG | ME | DG | DE | TG | TOTAL% |
|---|-----|-----|-----|------|-----|------|-----|--------|
| 0 | 1.7 | 2.2 | 0 | 78.7 | 0 | 18.3 | 0 | 100.9 |

Example 8

Preparation of material to be purified

Scale:

| | weight (g) |
|---|---|
| Coconut oil (from Liverpool Central Oils Ltd) | 1171 |
| Potassium hydroxide | 47.7 |
| Potassium carbonate | 119.2 |
| Sugar (powdered) | 763 |

The coconut oil was melted in an oil bath and potassium soaps prepared by the addition of the potassium hydroxide accompanied by vigorous agitation. The potassium carbonate was added and the temperature raised to 125°C over 0.5 hour. The sugar was added in increments and the mixture was allowed to react for 21 hours.

Analysis of crude material

| G | So | Su | MG | ME | DG | DE | TG | TOTAL% |
|---|---|---|---|---|---|---|---|---|
| 0 | 20.9 | 20.6 | MG+DG+TG = 23.4%, | | | ME+DE = 35.1 | | 100.0 |

Isolation of Coconut sucrose esters

Scale:

1 kg crude material + 6 $dm^3$ water

200 $cm^3$ glacial acetic acid

The solid was dispersed in the water and the acid added.  At 20°C an oil in water dispersion was formed and on cooling to 18°C, coagulation occurred but no separation.  The mixture was heated to 35°C and allowed to stand for several hours where upon the oil separated to the bottom of the flask.  The upper layer was discarded and water at 18°C added to the oily residue.  The mixture was stirred gently and then allowed to settle and the upper layer discarded.  The oil was dried on a Büchi evaporator.

Weight of organic material    703.5g

The organic material was then extracted two times using ethyl acetate in respective amounts at respective temperatures and for respective times of $4.2dm^3$, 6°C and 45 minutes; and $3dm^3$, 9°C and 30 minutes.  190.7g of purified product with the following analysis was obtained.

| G | FFA | Su | MG | ME | DG | DE | TG | TOTAL% |
|---|-----|-----|-----|------|-----|------|-----|--------|
| 0 | 1.1 | 5.0 | 0 | 67.1 | 0 | 21.1 | 0 | 94.3 |

Example 9

Preparation of material to be purified

Scale:

|                             | weight (g) |
|-----------------------------|-----------|
| Palm oil (from Pure Lard Ltd) | 1500 |
| Potassium hydroxide         | 61.1 |
| Potassium carbonate         | 152.7 |
| Sugar (powdered)            | 978 |

Soaps were prepared _in situ_ by the addition of potassium hydroxide to the melted palm oil and homogenising. The potassium carbonate was added and the mixture stirred for 0.5 hour. The temperature was raised to 125°C and the sugar was added in portions. The mixture was allowed to react for seven hours after the addition of the sugar.

Weight of crude material 2.654 kg

Analysis of crude material

| G | So | Su | MG | ME | DG | DE | TG | TOTAL% |
|---|-----|-----|-----|------|-----|-----|-----|--------|
| 2.4 | 28.7 | 21.7 | 9.5 | 19.7 | 6.8 | 9.3 | 1.6 | 99.7 |

## Isolation of Palm Oil Sucrose Esters

Scale:

663g crude material + 6 dm$^3$ water

133.6g glacial acetic acid

The crude product was dispersed in the water and the acid added slowly with stirring.

The mixture was stirred for 0.5 hour then the temperature slowly raised to 50°C (the material formed granules at 37°C and melted at 43-45°C). The lower aqueous layer was removed and the organic layer made up to the original volume with water. The mixture was stirred, heated to 50°C to effect separation and decanted. This procedure was repeated, heating to 80°C for the final wash. The organic layer was dried on the Büchi.

Weight of organic material    456.8g

The organic material was then extracted three times using ethyl acetate in respective amounts at respective temperatures and for respective times of $2.7dm^3$, 5°C and 30 minutes; $2.7dm^3$, 5°C and 30 minutes; and $2dm^3$, 17°C and 30 minutes. 85.7g of purified product with the following analysis was obtained.

| G | FFA | Su | MG | ME | DG | DE | TG | TOTAL% |
|---|-----|-----|-----|------|-----|------|-----|--------|
| 0 | 0.74 | 1.0 | 0.7 | 83.9 | 0.5 | 15.9 | 0 | 102.7 |

Example 10

Preparation of material to be purified

Scale:

weight (g)

    Part-hydrogenated rapeseed oil (from Pure Lard Ltd) 1114

    Potassium hydroxide                                 61.1

    Potassium carbonate                               113.4

    Sugar (powdered)                                    726

The potassium soaps were prepared by homogenising the melted oil and potassium hydroxide. The potassium carbonate was added and the mixture stirred to 0.5 hour. When the temperature of the mixture was 125°C the sugar was added in five increments over 1.5 hours. The mixture was then allowed to react for 4 hours.

Weight of crude material       1958.9g

Analysis of crude material

| G | So | Su | MG | ME | DG | DE | TG | TOTAL% |
|-----|------|------|------|------|------|-----|-----|--------|
| 2.1 | 27.0 | 24.5 | 14.3 | 17.8 | 4.0 | 8.2 | 2.0 | 99.9 |

Isolation of part-hydrogenated rapeseed oil sucrose esters

Scale

    1 kg crude product + 6 dm$^3$ water
    200 cm$^3$ glacial acetic acid

The solid was dispersed in the water and the acid added with stirring. The mixture was stirred for 0.5 hour and the temperature raise to 50°C (the organic phase melted at 43°C). The organic layer was separated and washed again by decantation. This procedure was repeated for a third time, the temperature being raised to 85°C before decantation. The oil was then dried using Büch equipment.

Weight of organic material        675.7g

The organic material was then extracted three times using ethyl acetate in respective amounts at respective temperatures and for respective times of $4dm^3$, 8°C and 30 minutes; $3dm^3$, 7°C and 90 minutes; and $2.5dm^3$, 18°C and 90 minutes. 170.0g of purified product with the following analysis was obtained.

| G | FFA | Su | MG | ME | DG | DE | TG | TOTAL% |
|---|-----|-----|-----|------|-----|------|-----|--------|
| 0 | 2.3 | 3.0 | 1.4 | 60.2 | 2.5 | 27.8 | 0.7 | 97.9 |

## Example 11

### Preparation of material to be purified

Scale:

Hydrogenated soya bean oil (from Pure Lard Ltd)        1114g

Potassium hydroxide        45.4g

Potassium carbonate        113.4g

Sugar (powdered)        726g

The hydrogenated soya bean oil was heated to 110°C and the potassium hydroxide added. The mixture was homogenised to form the potassium soaps and then the potassium carbonate was added and the mixture stirred for 0.5 hours while the temperature was raised to 125°C. The sugar was added in increments over 1 hour and the reaction mixture was then stirred for 5 hours.

Weight of crude material      1965.2g

Analysis of crude material

| G | So | Su | MG | ME | DG | DE | TG | TOTAL% |
|---|----|----|----|----|----|----|----|--------|
| 2.1 | 24.1 | 23.0 | 13.5 | 19.8 | 4.0 | 10.7 | 3.7 | 100.9 |

## Isolation of hydrogenated soya bean sucrose esters

### Scale

1 kg crude product + 6 dm$^3$ water

200 cm$^3$ glacial acetic acid

The solid was dispersed in water and the acid added slowly. The mixture was stirred for 0.5 hour at room temperature and then the temperature raised to 50°C (separation began at 35°C and was complete at 42°C). The lower aqueous layer was removed by siphoning and the organic layer was reconstituted to its original volume with water and stirred for 0.5 hour. The temperature was raised to 50°C and the water removed by siphoning. The organic layer was once again reconstituted to its original volume with water and stirred for 0.5 hour and then heated to 80°C without stirring. On cooling slightly, the aqueous layer was removed and discarded. The organic layer was Büchi dried.

Weight of organic material    677.7g

The organic material was then extracted three times using ethyl acetate in respective amounts at respective temperatures and for respective times of $4dm^3$, 7°C and 30 minutes; $3dm^3$, 9°C and 30 minutes; and $3dm^3$, 17°C and 30 minutes. 132.3g of purified product with the following analysis was obtained.

| G | FFA | Su | MG | ME | DG | DE | TG | TOTAL% |
|---|-----|-----|-----|------|-----|------|-----|--------|
| 0 | 1.5 | 3.1 | 0 | 75.3 | 0 | 20.0 | 0 | 99.9 |

## Example 12

### Preparation of material to be purified

### Scale:

| | |
|---|---|
| Hardened tallow (from Kelanco Ltd) | 3000g |
| Potassium hydroxide | 122g |
| Potassium carbonate | 305g |
| Sugar (powdered) | 1956g |

The hardened tallow was melted in an oil bath and the potassium hydroxide added. The mixture was homogenised and the potassium carbonate was added. The mixture was stirred for 0.5 hour, and then the sugar was added in portions. After the additon of the sugar the reaction mixture became so viscous that it could not be stirred adequately. The mixture was stirred intermittently by hand at a temperature of 110°C for 5 hours, after the addition of the sugar.

Weight of crude material 5.283kg

Analysis of crude material

| G | So | Su | MG | ME | DG | DE | TG | TOTAL% |
|---|----|----|----|----|----|----|----|--------|
| 1.7 | 25.3 | 24.4 | 10.8 | 16.1 | 7.4 | 8.5 | 6.0 | 100.2 |

## Isolation of hardened tallow sucrose esters

### Scale

1 kg crude material + 6 $dm^3$ water

200 $cm^3$ glacial acetic acid

The crude material was dispersed in water and the glacial acetic acid was added. The mixture thickened to such a point that it required manual stirring. The temperature was raised to 65°C in order to melt the organic layer and the water was removed. This procedure was twice repeated and the organic layer was dried using Büchi equipment.

Weight of organic material 666.8g

The organic material was then extracted three times using ethyl acetate in respective amounts at respective temperatures and for respective times of 8dm$^3$, 8°C and 30 minutes; 3dm$^3$, 7°C and 30 minutes; and 8dm$^3$, 23°C and 30 minutes.  233g of purified product with the following analysis was obtained.

| G | FFA | Su | MG | ME | DG | DE | TG | TOTAL% |
|---|-----|----|----|----|----|----|----|--------|
| 0 | 8.5 | 2.8 | 9.2 | 33.1 | 7.9 | 20.6 | 12.5 | 96.7 |

## Example 13

### Preparation of material to be purified

### Scale:

| | |
|---|---|
| Palm Stearin – origin unknown | 1114g |
| Potassium hydroxide | 45.4g |
| Potassium carbonate | 113.4g |
| Sugar (powdered) | 726g |

The soaps were prepared by homogenising the palm stearin and potassium hydroxide at 105°C.  The potassium carbonate was added and the mixture stirred for 0.5 hour while the temperature was raised to 125°C.  The powdered sugar was added in 6 increments over 3 hours and the mixture was stirred for a further 2 hours.

Weight of crude material          1959.1g

0065390

Analysis of crude material

| G | So | Su | MG | ME | DG | DE | TG | TOTAL% |
|---|---|---|---|---|---|---|---|---|
| 0 | 22.8 | 11.5 | 12.0 | 24.6 | 7.0 | 10.6 | 10.0 | 98.5 |

## Isolation of palm stearin sucrose esters

## Scale

1 kg crude material + 6 $dm^3$ water

200 $cm^3$ glacial acetic acid + 200 $cm^3$ water

The solid was dispersed in the water and the acid solution was added with stirring. The mixture was stirred for 20 minutes and then the temperature was raised to 50°C (the organic material melted 45 to 47°C). The aqueous layer was decanted and the organic layer washed by a further 2 decantations. The residue was dried on a rotary vacuum evaporator.

Weight of organic material    675.9g

The organic material was then extracted three times using ethyl acetate in respective amounts at respective temperatures and for respective times of 4$dm^3$, 9°C and 90 minutes; 3$dm^3$, 14°C and 90 minutes; and 2$dm^3$, 14°C and 90 minutes. 175g of purified product with the following analysis was obtained.

| G | FFA | Su | MG | ME | DG | DE | TG | TOTAL% |
|---|-----|-----|-----|------|-----|------|-----|--------|
| 0 | 3.3 | 3.0 | 0.7 | 53.2 | 1.9 | 22.8 | 0.3 | 85.2* |

* gas chromatography shows evidence of higher esters

CLAIMS

1.   A process for purifying a sucrose ester-containing material obtained by base-catalysed transesterification of sucrose with a fatty acid ester, which process comprises the steps of:

    (i)    acidifying an aqueous dispersion of the material to between pH 4 and 7 to give an aqueous phase and an organic phase;

    (ii)    at or above the melt temperature of the organic phase, recovering the organic phase;

    (iii)   mixing the organic phase with ethyl acetate containing 0 to 4% water, to give at a temperature of 0 to 20°C a liquid phase and a solid phase; and

    (iv)    recovering the solid phase as a sucrose ester-rich product.

2.     A process according to claim 1, wherein the fatty acid profile of the fatty acid ester includes at least 50% acids with 16 or more carbon atoms.

3.     A process according to claim 1 or 2, wherein the fatty acid profile of the fatty acid ester includes 5 to 50% acids with unsaturation.

4.     A process according to any preceding claim, wherein

0065390

in step (i) the dispersion contains 5 to 15% of the said
material.

5.      A process according to any preceding claim, wherein
the dispersion in step (i) is at below the melt temperature
specified for step (ii).

6.      A process according to any preceding claim, wherein
the pH in step (i) is around pH 4.5 when using a weak organic
acid or approaching pH 7 when using a strong mineral acid.

7       A process according to any preceding claim, wherein
in step (iii) the organic phase:ethyl acetate ratio is
between 1:10 and 1:3, expressed as $kg:dm^3$.

8.      A process according to claim 6, wherein in step (iii)
the organic phase:ethyl acetate ratio is between 1:7 and 1:5,
expressed as $kg:dm^3$.

9       A process according to any preceding claim, wherein
in step (iii) two to four extractions are carried out using a
higher temperature at each extraction.

10      A process according to any preceding claim, wherein
the recovered solid phase of step (iv) is obtained as a
free-flowing powder which meets the herein-defined EEC
Specification No E473 for sucrose esters.

## EUROPEAN SEARCH REPORT

**European Patent Office**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | FR-A-2 032 778 (DAI-ICHI) * Page 6, lines 17-38; page 7; page 8; page 9; page 16 * & GB - A - 1 295 721 | 1-9 | C 07 H 13/06 |
| Y | GB-A-1 180 103 (STATE OF NEBRASKA) * Page 6, example 6; page 7, example 10 * | 1 | |
| Y | GB-A-1 018 554 (LEDOGA) * Page 1, lines 82-90; page 2, lines 73-80; page 2, lines 121-124 * | 1 | |
|  |  |  | **TECHNICAL FIELDS SEARCHED (Int. Cl. ³)** |
|  |  |  | C 07 H 13/00 |

The present search report has been drawn up for all claims

| Place of search | Date of completion of the search | Examiner |
|---|---|---|
| THE HAGUE | 19-08-1982 | VERHULST W. |

EPO Form 1503, 03.82

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document